Europäisches Patentamt

(19) European Patent Office

Office européen des brevets

(11) Publication number : **0 411 970 B1**

# (12) EUROPEAN PATENT SPECIFICATION

(45) Date of publication of patent specification :
**04.08.93 Bulletin 93/31**

(51) Int. Cl.[5] : **A61L 2/10, A61L 2/18**

(21) Application number : **90401441.2**

(22) Date of filing : **30.05.90**

(54) **Sterilization of containers by means of hydrogen peroxide, peracids, and u.v. radiation.**

(30) Priority : **26.06.89 US 371152**

(43) Date of publication of application :
**06.02.91 Bulletin 91/06**

(45) Publication of the grant of the patent :
**04.08.93 Bulletin 93/31**

(84) Designated Contracting States :
**AT BE CH DE DK ES FR GB GR IT LI LU NL SE**

(56) References cited :
**WO-A-80/01457**
**GB-A- 1 570 492**

(56) References cited :
**CHEMICAL ABSTRACTS, vol. 77, no. 25, 1972,
Columbus, OH (US); E.L. POWERS et al., p.
153, no. 161570r**
**CHEMICAL ABSTRACTS, vol. 70, no. 5, 1969,
Columbus, OH (US); C.O. DOUDNEY, p. 1778,
no. 17785c**

(73) Proprietor : **FMC Corporation
2000 Market Street
Philadelphia Pennsylvania 19103 (US)**

(72) Inventor : **Wang, James J.
7041 Blue Hill Drive
San Jose, CA 95129 (US)**

(74) Representative : **Rodhain, Claude et al
Cabinet Claude Rodhain 30, rue la Boétie
F-75008 Paris (FR)**

EP 0 411 970 B1

Jouve, 18, rue Saint-Denis, 75001 PARIS

## Description

This invention relates to a method for sterilizing articles, liquids and the like and treated products thereof. More particularly, this invention relates to the sterilization of various surfaces, particularly packaging materials, with a combination of reagents and ultraviolet light. Still more particularly this invention is directed to the sterilization of plastic food containers by contacting them with hydrogen peroxide, a peracid, and ultraviolet light.

Sterilization of articles such as plastic food containers or membranes for medical treatment is a prerequisite for aseptic packaging, treatment, and processing concepts. Traditionally, plastic containers, for example, are sterilized by means of $H_2O_2$ solutions at elevated temperatures and sufficient contact times in order to achieve surface sterilization. Thus, $H_2O_2$ solutions of about 30-35% at temperatures of about 80-85°C and contact times of at least about 20 seconds have been conventionally employed for this purpose.

The prior art has shown that the concentration of $H_2O_2$ can be reduced to about 0.25 to 5% when other lethal mechanisms are employed simultaneously. Results from U.S. Patent 4,289,728, for example, indicate that a 4+ log cycle reduction in the number of B. subtilis spores can be achieved when such spore suspension in 0.25% $H_2O_2$ is subjected to 30 seconds of U.V. irradiation, followed by heating to 85°C for 60 seconds. However, this method thus entails 90 seconds per treatment. Also, British Patent 1,570,492 demonstrates that flat packaging material (polystyrene strip) can be sterilized by means of a sterilant comprised of $H_2O_2$ (>20%) and $CH_3COOOH$ (0.01-0.5%) in an aqueous solution. Furthermore, this patent teaches that as much as a 6 log reduction in the number of B. subtilis spores can be achieved as the solution of $H_2O_2/CH_3COOOH$ is applied to the surface of the packaging material, followed by hot air (65-86°C) treatment for an additional 2-12 seconds.

Such high levels of $H_2O_2$, acids, and temperature, together with a relatively long contact time, are necessary to meet effective surface sterilization as demanded by microbiological standards of aseptic packaging operations. However, since the resultant high levels of $H_2O_2$ residue might end up in the packaged product, the food industry, for example, is continuously looking for better control and/or alternatives to cope with such a problem. In fact, $H_2O_2$ residues in foods have been restricted by regulation to very low levels (0.5 ppm, that is, 0.5 mg of $H_2O_2$/1000 cc of $H_2O$). Therefore, efforts have been made to reduce the concentration of $H_2O_2$ being used in these operations, while at the same time maintaining or reducing the temperatures and contact time required to achieve adequate sterilization.

Moreover, configuration of packaging material (flat vs. preformed containers) also exhibits a great deal of impact on the sporicidal effect of any sterilization treatment. In the case of sterilization of preformed packaging containers, for example, uneven distribution of sterilant and other sterilizing means results in tremendous differences in achieving satisfactory sterility. Therefore, the methods and techniques suggested by the above-mentioned patents are not really considered to be practical or commercially effective compared to the present invention disclosed herein.

In accordance with the present invention there is provided an improved method, and resulting sterilized products thereof, for the sterilization of articles such as packaging material, membranes used in medical treatment, as well as plastic food containers, which method comprises contacting said articles with a combination of agents comprising hydrogen peroxide, a peracid, and ultraviolet (U.V.) light, that is an ultraviolet irradiated solution of hydrogen peroxide and peracid in amounts sufficient to sterilize said articles or liquids. When treated with these agents in a manner described in further detail below, there is achieved the highly effective sterilization of such articles at low concentrations of hydrogen peroxide, and at ambient temperatures and short contact times. More particularly, in contrast with the prior art the present method provides the advantages of (1) lower concentrations of $H_2O_2$ (for example, about 15-25% vs. as much as 30-35%); (2) better sporicidal effect (for example, >6.0 log reduction); (3) ambient temperatures; and (4) shorter U.V. contact time (for example, about 8-12 seconds).

When the articles to be sterilized are treated by the combination of agents described herein, there are obtained several advantages over known prior art methods. Thus, for example, this treatment is particularly effective not only in the sterilization of flat articles or surfaces but also preformed materials containing angled surfaces, corners, and the like which are much more difficult to sterilize by known means. In addition, this process is highly effective against dry spores, as contrasted with the more readily susceptible wet spores described in the art. Moreover, this treatment can be carried out entirely at ambient temperatures without the need for application of any preceding or concurrent heating steps to activate the chemicals. Finally, the combination of agents defined herein results in synergistic effects beyond what is accountable by the additive effects of each of these agents alone.

This process may be carried out in a generally known manner in which the article to be sterilized, as for example preformed polypropylene cups, is subjected to the treatment of a sterilizing medium comprising an aqueous solution of $H_2O_2$ and $CH_3COOOH$ by means of spraying. The concentration of $H_2O_2$ and $CH_3COOOH$ desirably lies within the range of about 15 to 25%, preferably about 20-25%, and 0.01 to 1%, preferably about 0.5-1.0%, respectively, by weight of the aqueous spray solution. Spraying sterilant into the container is desirably carried out at ambient temperature, preferably about 20-30°C, although somewhat higher temperatures are desirable when spores are present; and the container is concurrently, or less desirably, sequentially irradiated by U.V. light having a wavelength of less than about 300 nm. The spraying time is desirably for about 0.5 to 1 second, but may be varied depending upon the size and shape of the container, while the irradiation time is desirably for a period of from about 5 to 20 seconds, preferably about 8-12 seconds.

Although the U.V. radiation employed may be as high as 300 nm, it is preferred that it be in the range of from about 200 to 280 nm, and most preferably about 254 nm. Generally speaking, the higher the radiation energy, the better the sporicidal effect. Also, at higher energies the time period to achieve sterility can be shorter. The energy received on the surface of packaging material is a major contributing factor to surface sterilization. In addition, container configuration also plays a vital role in this respect. For example, some containers may have deep bodies and narrow mouths, while others may have shallow bodies and wide mouths. In such cases the amount of energy should be routinely adjusted somewhat in accordance with known art methods. Other factors which should also be considered in determining the amount of U.V. employed include the distance between the lamp and the object, the type of microorganism, and the like.

The peracid employed is preferably peracetic acid, but other peroxyacids may likewise be employed in this novel process as long as they are safe for human consumption and are compatible with the food or other material being sterilized. These peracids are characterized by their low pH, and germicide activity, as well as the safety of their end products.

While the concentration of hydrogen peroxide and peracid, as stated above, is desirably within the range of about 15 to 25% and 0.01 to 1.0% respectively, by weight of the aqueous spray solution, when the microorganism is present in the form of spores, particularly dry spores, it is preferred for reasons described below that the higher concentration of these ranges be used, that is, about 20 to 25% and 0.5 to 1.0% by weight, respectively. This is particularly true of the upper ranges of the peracid concentration, which, as shown by Tables 2 and 3 below, provide significant increases in sporicidal effect with small increases in concentration of the peracid.

Following sterilization the articles may then desirably be subjected to sterile hot air, for example at temperatures of at least about 70°C, and preferably from about 70-120°C, primarily for the purpose of dissipating any remaining $H_2O_2$. Higher temperatures may be employed when the nature of the article permits in order to shorten the overall processing time.

The physical means by which the process is carried out is not critical, and may include the use of sprayers, preferably ultrasonic sprayers; heaters; U.V. radiation means; and like conventional means known to those skilled in the art, including means by which containers such as food containers are contacted with the combination of reagents and U.V. light on a conveyor belt or like means. Ultrasonic sprayers are preferred because they provide thin, uniform coatings of sterilant without leaving droplets on the surface.

This invention is applicable to a wide array of microorganisms, particularly those where spores are present. Included are such organisms as bacteria, molds, and the like, as well as such other organisms as protozoa or viruses.

In dealing with surface sterilization, dry spores should preferably be the main target organism, and therefore should be considered as a better control point in determining the effectiveness of the process. In other words, the measure of true lethality should more preferably be based on using dry bacterial spores as the indicator organism instead of wet forms of bacterial spores in the sterilization process of packaging materials. The reason for this is that dry spores have been found to be up to two times more resistant to the sporicidal effects of $H_2O_2$ than wet spores. See Leaper, Journal of Food Technology, Vol. _19_, pp. 635-702. (1984). By contrast, it is evident from the conditions in the above-cited U.S. and British patents that the more susceptible wet spores are the target organisms when in fact only dry packaging materials, and thus dry spores, are supplied by manufacturers.

The invention will now be illustrated by the following examples.

EXAMPLES

In order to illustrate the lethal effect of the combined treatment defined by this process, survival rates of dried spores of _B. subtilis_ var. _niger_ ATCC 9372 were established (Table 1). For each test, two test samples were used. In certain runs, as discussed further below, elevated temperatures such as used by the prior art were employed for comparison. Spores were uniformly deposited onto the inner surface of 113,4 g (4 oz) polypropylene cups and the surface dried for at least 12 hours in an electrical dryer to ensure dry spores. The initial spore load for each cup was $1 \times 10^7$. The U.V. lamp, fitted in combination with an ultrasonic spray device, was positioned 2,54 cm (1 inch) above the top of each tapered 113,4 g (4-oz). container 3,8 cm (1.5") deep by 6,99 cm (2.75") wide at its maximum diameter). The measured U.V. intensity (mW/cm²) (in each cup) was 7.8 at the bottom; 3.0 at the inner edge (top); and 2.4 on the sides. Each cup was subjected to sterilization treatment with U.V. light (254 nm) and an aqueous spray solution of $H_2O_2$ and $CH_3COOOH$ under conditions described in the tables below, using an ultrasonic spraying device; the spraying time was 0.9 sec. at ambient temperature.

As a further test of sporicidal properties vs. time of exposure to U.V. irradiation, Table 2 illustrates the sporicidal capability by using the same physical arrangement and a combined treatment of $H_2O_2/CH_3COOOH$ and U.V. irradiation on dry spores. The spore-killing effect was increased with an increase in time of U.V. exposure. A similar result was also observed with an increase in the concentration of $CH_3COOOH$ (from 0.07% to 0.12%) while keeping the $H_2O_2$ concentration in the test solution constant. Such results can be further demonstrated by Table 3, wherein as much as a 6.5 log cycle reduction in the number of bacterial spores resulted from a test solution consisting of varied concentrations of $CH_3COOOH$ and 22.5% $H_2O_2$, together with 10 seconds of U.V. irradiation at ambient temperatures.

In each case, sterile, dry, hot air (70-120°C) is used as a means to dissipate these chemicals after sterilization and prior to the filling operation in the sterile chamber. The containers thus leave the sterilizing/dry station both dry and sterile and, in the case of foodstuffs, may thereafter be filled in a filling station, closed, and then sealed. The sterilized article or product is then ready for commercial distribution.

## TABLE 1

### Sporicidal Effect of $H_2O_2/CH_3COOOH/U.V.$[2] on B. subtilis[1] in Preformed Packaging Containers

| Example | H₂O₂ (%) | CH₃COOOH (%) | UV (Sec) | Temp (°C) | Log Reduction in Bacterial Spores |
|---|---|---|---|---|---|
| 1 | 22.5 | --- | --- | 25 | 1.6 |
| 2 | 22.5 | --- | 10 | 25 | 2.8 |
| 3 | 22.5 | --- | 10 | 80 | 2.4 |
| 4 | 22.5 | --- | --- | 80 | 3.6 |
| 5 | --- | 0.6 | --- | 25 | 1.7 |
| 6 | --- | 0.6 | 10 | 25 | 4.2 |
| 7 | --- | 0.6 | 10 | 80 | 4.7 |
| 8 | --- | 0.6 | --- | 80 | 2.8 |
| 9 | 22.5 | 0.6 | -- | 25 | 2.0 |
| 10 | 22.5 | 0.6 | 10 | 25 | 6.5 |

1. Initial organism load = $1 \times 10^7$ (or log # = 7.0) of B. subtilis var. niger (ATCC 9372) on a polypropylene cup with 113,4 g (4 oz.) capacity.

2. U.V. radiation = 254 nm for indicated times.

## TABLE 2

Sporicidal* Effect Using Combined Treatment[a] of
Constant Concentration of $H_2O_2$[b], With Peracetic
Acid and U.V. Radiation[c]

| Example | Peracid Conc. (wt %) | U.V. Radiation Time (sec.) | Log Reduction in Bacterial Spores[d] |
|---|---|---|---|
| 11 | 0.07 | 10 | 3.0 |
| 12 | 0.07 | 15 | 3.1 |
| 13 | 0.07 | 20 | 3.3 |
| 14 | 0.12 | 10 | 3.6 |
| 15 | 0.12 | 15 | 3.8 |
| 16 | 0.12 | 20 | 4.3 |

a. Temperature = ambient (°C)

b. $H_2O_2$ Conc. = 22.5 wt. %

c. U.V. radiation = 254 nm for indicated times

d. Initial count = 1 x $10^7$/cup

* B. subtilis var. niger (ATCC 9372) on 113,4 g (4 oz.) polypropylene cups

EP 0 411 970 B1

## TABLE 3

Sporicidal* Effect Using Combined Treatment[a]
of Varied Concentrations of Peracetic Acid,
With Hydrogen Peroxide[b] and U.V. Radiation[c]

| Example | Peracid Conc. (wt %) | Log Reduction in Bacterial Spores[d] |
|---------|----------------------|--------------------------------------|
| 17 | .07 | 3.0 |
| 18 | .12 | 3.4 |
| 19 | .28 | 5.3 |
| 20 | .42 | 6.0 |
| 21 | .60 | 6.5 |

a.  Temperature = ambient ($^\circ$C)

b.  $H_2O_2$ Conc. = 22.5 wt. %

c.  U.V. radiation = 254 nm for 10 sec.

d.  Initial count = $1 \times 10^7$/cup

*   B. subtilis var. niger (ATCC 9372) on 113,4 g (4 oz.) polypropylene cups

From the results shown in the above tables, the following will be seen: Table 1 shows from the number of survivors that the sporicidal effect from the combined treatment with $H_2O_2$, peracid, and U.V. radiation (Example 10) far exceeds, by several-fold, the effect obtained by the prior art $H_2O_2$ or peracid alone, or in combination, or $H_2O_2$ and U.V. Moreover, the prior art expedient of using elevated temperatures (80°C) surprisingly gave inconsistent and generally poorer results. Thus, the novel method defined herein unexpectedly results in a synergistic sporicidal effect which could not be predicted from the additive results of the closest prior art methods.

Table 2 demonstrates that within moderate ranges, small increases in peracid concentration and radiation time can result in further improvements in sporicidal effect, given constant $H_2O_2$ concentration. Table 3 shows similar benefits can be obtained by varying the peracid concentration alone.

## Claims

1.  A method of sterilizing microorganism-containing articles characterized by treating said articles or liquids with ultraviolet light and a solution of hydrogen peroxide and a peracid in amounts sufficient to sterilize said articles or liquids.

2.  The method of claim 1 characterized in that the microorganisms are dry bacterial spores.

3.  The method of claim 1 characterized in that the articles are shaped food containers.

6

4. The method of claim 1 characterized in that the wavelength of the ultraviolet radiation is below 300 nm.

5. The process of claim 4 characterized in that the ultraviolet radiation is from 200 to 280 nm.

6. The process of claim 1 characterized in that the ultraviolet light contact time is from 5 to 20 seconds.

7. The method of claim 1 characterized in that the peracid is peracetic acid.

8. The method of claim 1 characterized in that the concentration of hydrogen peroxide is in the range of 15-25% by weight of the solution.

9. The method of claim 1 characterized in that the concentration of peracid is in the range of from 0.01-1% by weight of the solution.

10. The method of claim 1 characterized in that the sterilization is carried out at ambient temperatures.

11. The method of claim 1 further characterized in that subsequent to sterilization the said articles are dried at elevated temperatures of at least 70°C.

12. Food packaging material, or liquids, when sterilized in accordance with the method of claim 1.


## Patentansprüche

1. Verfahren zum Sterilisieren von Gegenständen, die Mikroorganismen enthalten, dadurch gekennzeichnet, daß man die Gegenstände oder Flüssigkeiten mit ultraviolettem Licht und einer Lösung aus Wasserstoffperoxid und einer Persäure in einer zum Sterilisieren der Gegenstände oder Flüssigkeiten ausreichenden Menge behandelt.

2. Verfahren nach Anspruch 1, dadurch gekennzeichnet, daß die Mikroorganismen trockene bakterielle Sporen sind.

3. Verfahren nach Anspruch 1, dadurch gekennzeichnet, daß die Gegenstände geformte Lebensmittelbehälter sind.

4. Verfahren nach Anspruch 1, dadurch gekennzeichnet, daß die Wellenlänge der ultravioletten Strahlung unterhalb 300 nm liegt.

5. Verfahren nach Anspruch 4, dadurch gekennzeichnet, daß die ultraviolette Strahlung zwischen 200 und 280 nm liegt.

6. Verfahren nach Anspruch 1, dadurch gekennzeichnet, daß die Kontaktzeit mit dem ultravioletten Licht 5 bis 20 Sekunden beträgt.

7. Verfahren nach Anspruch 1, dadurch gekennzeichnet, daß die Persäure Peressigsäure ist.

8. Verfahren nach Anspruch 1, dadurch gekennzeichnet, daß die Wasserstoffperoxidkonzentration im Bereich von 15 bis 25 Gew.-% der Lösung liegt.

9. Verfahren nach Anspruch 1, dadurch gekennzeichnet, daß die Persäurekonzentration im Bereich von 0,01 bis 1 Gew.-% der Lösung liegt.

10. Verfahren nach Anspruch 1, dadurch gekennzeichnet, daß die Sterilisation bei Umgebungstemperaturen ausgeführt wird.

11. Verfahren nach Anspruch 1, zusätzlich gekennzeichnet, daß nach der Sterilisation die Gegenstände bei erhöhten Temperaturen von mindestens 70°C getrocknet werden.

12. Verpackungsmaterial für Lebensmittel oder Flüssigkeiten, sterilisiert gemäß dem Verfahren von Anspruch 1.

**Revendications**

1. Procédé pour stériliser des articles contenant des micro-organismes, caractérisé en ce qu'on traite lesdits articles ou liquides par des rayons ultraviolets et une solution de peroxyde d'hydrogène ainsi qu'un peracide en quantités suffisantes pour stériliser lesdits articles ou liquides.

2. Procédé selon la revendication 1, caractérisé en ce que les micro-organismes sont des spores bactériennes sèches.

3. Procédé selon la revendication 1, caractérisé en ce que les articles sont des récipients alimentaires façonnés.

4. Procédé selon la revendication 1, caractérisé en ce que la longueur d'onde des rayons ultraviolets est inférieure à 300 nm.

5. Procédé selon la revendication 4, caractérisé en ce que la longueur d'onde des rayons ultraviolets est comprise entre 200 et 280 nm.

6. Procédé selon la revendication 1, caractérisé en ce que le temps de contact des rayons sultraviolets est de 5 à 20 secondes.

7. Procédé selon la revendication 1, caractérisé en ce que le peracide est l'acide peracétique.

8. Procédé selon la revendication 1 caractérisé en ce que la concentration en peroxyde d'hydrogène est comprise dans la gamme allant de 15 à 25% du poids de la solution.

9. Procédé selon la revendication 1, caractérisé en ce que la concentration en peracide est comprise dans la gamme allant de 0,01 à 1% du poids de la solution.

10. Procédé selon la revendication 1, caractérisé en ce qu'on effectue la stérilisation aux températures ambiantes.

11. Procédé selon la revendication 1, caractérisé en outre en ce que, après la stérilisation on sèche lesdits articles à des températiures élevées, au moins égales à 70°C.

12. Matériau pour emballage alimentaire, ou liquides ayant été stérilisés suivant le procédé de la revendication 1.